# EUROPEAN PATENT SPECIFICATION

(11) **EP 2 869 070 B1**
(45) Date of publication and mention of the grant of the patent: **28.03.2018**
(21) Application number: 14190568.7
(22) Date of filing: 28.10.2014
(51) Int. Cl.: G01N 33/564, G01N 33/68, A61B 5/145, C12Q 1/68, A61B 5/00

(54) **Method, system and computer programme product for assisting diagnosis of rheumatoid arthritis**
Verfahren, System und Computerprogrammprodukt zur Unterstützung der Diagnose von rheumatoider Arthritis
Procédé, système et produit de programme informatique pour faciliter le diagnostic de la polyarthrite rhumatoïde

(30) Priority: 28.10.2013 JP 2013223592
(43) Date of publication of application: 06.05.2015
(73) Proprietor: SYSMEX CORPORATION, Kobe-shi, Hyogo 651-0073 (JP); Shinko Medical Corporation, Kobe-shi, Hyogo 651-0072 (JP)
(72) Inventor: Miyamoto, Yoshiaki, Kobe-shi, Hyogo 651-0073 (JP); Okazawa, Takahiro, Kobe-shi, Hyogo 651-0073 (JP); Kumagai, Shunichi, Amagasaki-shi, Hyogo 661-0953 (JP)
(74) Representative: Paemen, Liesbet R.J.

(56) References cited:
- WO-A1-2011/025962
- WO-A2-2005/029091
- WO-A2-2007/123976
- WO-A2-2010/053587
- US-A1- 2012 121 594
- "IBMS poster-Thursday ED - Karsenty Gerard; MacDougald Ormond; Rosen Clifford", BONE, PERGAMON PRESS., OXFORD, GB, vol. 40, no. 6, 1 June 2007 (2007-06-01), pages S267-S313, XP022130898, ISSN: 8756-3282
- Y. HATANO ET AL: "Macrophage inflammatory protein 1 alpha expression by synovial fluid neutrophils in rheumatoid arthritis", ANNALS OF THE RHEUMATIC DISEASES, vol. 58, no. 5, 1 May 1999 (1999-05-01), pages 297-302, XP055178013, ISSN: 0003-4967, DOI: 10.1136/ard.58.5.297
- JEFFREY H RUTH ET AL: "Role of Macrophage Inflammatory Protein-3[alpha] and Its Ligand CCR6 in Rheumatoid Arthritis", LABORATORY INVESTIGATION, vol. 83, no. 4, 1 April 2003 (2003-04-01), pages 579-588, XP055178014, ISSN: 0023-6837, DOI: 10.1097/01.LAB.0000062854.30195.52
- Anonymous: "MIPs - Genedirex", , 9 December 2011 (2011-12-09), XP055178036, Retrieved from the Internet: URL:http://web.archive.org/web/20111209001 201/http://www.genedirex.com/?page_id=880 [retrieved on 2015-03-20]
- RANTAPÄÄ-DAHLQVIST SOLBRITT ET AL: "Antibodies against cyclic citrullinated peptide and IgA rheumatoid factor predict the development of rheumatoid arthritis", ARTHRITIS & RHEUMATISM, WILEY, US, vol. 48, no. 10, 1 October 2003 (2003-10-01), pages 2741-2749, XP002333657, ISSN: 0004-3591, DOI: 10.1002/ART.11223
- VOSSENAAR E R ET AL: "CITRULLINATED PROTEINS: SPARKS THAT MAY IGNITE THE FIRE IN RHEUMATOID ARTHRITIS", ARTHRITIS RESEARCH AND THERAPY, BIOMED CENTRAL, LONDON, GB, vol. 6, no. 3, 1 January 2004 (2004-01-01) , pages 107-111, XP001207072, ISSN: 1478-6354, DOI: 10.1186/AR1184

## Description

### TECHNICAL FIELD

The present invention relates to a method for assisting diagnosis of rheumatoid arthritis. The present invention also relates to a system for providing information that assists diagnosis of rheumatoid arthritis and a computer program product for enabling a computer to carry out provision of the information.

### BACKGROUND ART

Rheumatoid arthritis is a systemic autoimmune disease of which the main clinical symptom is arthritis. Rheumatoid arthritis causes not only arthralgia but also destruction of cartilage and bone. It is known that joint destruction resulting from rheumatoid arthritis rapidly proceeds within 2 years from the onset. Meanwhile this period of within 2 years from the onset is regarded as the period (so-called window of opportunity) when therapeutic treatment is most required for remission induction because the susceptibility to antirheumatic therapy is high. Therefore early and accurate diagnosis is important for rheumatoid arthritis therapy.

Currently rheumatoid arthritis is diagnosed on the basis of subjective symptoms such as arthralgia, observation by physicians such as the extent of joint swelling, tests of serologic factors or radiography. Particularly the tests of serological factors are simple and objective, and thus are extremely useful for assisting diagnosis. In the tests, autoantibodies in serum such as rheumatoid factor (RF) and anti-cyclic citrullinated peptide antibody (anti-CCP antibody: ACPA) are quantified. Results of the measurements are regarded as important items in the ACR/EULAR diagnosis criteria proposed by The American College of Rheumatology (ACR) and The European League Against Rheumatism (EULAR).

RF is positively detected in about 80% of rheumatoid arthritis patients while it is negatively detected in the rest of about 20% of the patients. Therefore diagnosis of rheumatoid arthritis cannot be made only with the test on RF. In addition, RF may be positively detected in healthy subjects or patients with other autoimmune diseases such as systemic lupus erythematosus. To the contrary, anti-CCP antibody is rarely detected in autoimmune diseases other than rheumatoid arthritis. In addition, being detected in patients with rheumatoid arthritis at early stages, anti-CCP antibody can be regarded as a marker having better classification ability of rheumatoid arthritis than RF.

However, the test of anti-CCP antibody has a sensitivity of only around 70%. Thus a subject who has been determined to be negative for anti-CCP antibody may be put into a follow-up status without definitive diagnosis on whether the subject has rheumatoid arthritis or other autoimmune diseases associated with joint abnormality. However if the subject actually has rheumatoid arthritis, the chance of administering therapeutic treatment during the window of opportunity is missed. Therefore there is a need in clinical practice for a method for determining whether or not a subject who has been determined to be negative for anti-CCP antibody has rheumatoid arthritis.

In recent years, cytokines have been attracting attention as novel markers for assisting diagnosis of rheumatoid arthritis. For example, WO 2005/029091 discloses certain combinations of cytokines which are found to be increased in patients with various autoimmune diseases including rheumatoid arthritis by exhaustive analysis of cytokines. WO 2005/064307 discloses a method for assessing the presence or absence of rheumatoid arthritis by combining the measured amounts of anti-CCP antibody and IL-6 by using a statistical classification algorithm. In the classification methods disclosed in WO 2005/029091 and WO 2005/064307, healthy subjects or patients with osteoarthritis are used as a control for patients with rheumatoid arthritis.

There have been reports suggesting the correlation between chemokines and rheumatoid arthritis. For example, Hatano Y. et al., Ann Rheum Dis 1999; 58: 297-302 disclose that the concentration of MIP-1α (macrophage inflammatory protein-1α) in synovial fluid is higher in patients with rheumatoid arthritis than in patients with osteoarthritis. Ruth J.H. et al., Lab Invest 2003; 83: 579-588 discloses that the concentration of MIP-3α (macrophage inflammatory protein-3α) in synovial fluid is higher in patients with rheumatoid arthritis than in patients with osteoarthritis.

### SUMMARY OF THE INVENTION

It appears that the above-mentioned references disclose that various cytokines and chemokines can serve as novel markers replacing autoantibodies for determination of rheumatoid arthritis. However, all of the markers disclosed in the above references relate to classification of patients with rheumatoid arthritis and healthy subjects or patients with osteoarthritis. Healthy subjects do not have joint abnormality in the first place and thus cannot be a subject of diagnosis of rheumatoid arthritis. Although joint abnormality is seen in osteoarthritis, the cause thereof is not autoimmune abnormality but degeneration or damage of joints due to aging or obesity. In addition, osteoarthritis can be easily differentiated from rheumatoid arthritis by current examinations such as radiography.

As described above, at the current moment no marker has been found that can distinguish rheumatoid arthritis, particularly anti-CCP antibody-negative rheumatoid arthritis from other autoimmune diseases associated with joint abnormality. US 2012/121594 A1 discloses a method for assisting diagnosis of rheumatoid arthritis, comprising steps of: measuring at least one chemokine in a blood sample derived from a subject and obtaining a concentration of the chemokine in the blood sample and comparing the concentration of the chemokine with a first threshold. Thus an object of the present invention is to find a novel marker which can distinguish as described above and to provide a method for assisting diagnosis of rheumatoid arthritis by using the marker.

The inventors of the present invention have made intensive studies and, as a result, found that based on the results of measurement of MIP-1α and MIP-3α in blood samples, rheumatoid arthritis, particularly anti-CCP antibody-negative rheumatoid arthritis can be distinguished from other autoimmune diseases associated with joint abnormality. Thus, the inventors have completed the present invention as defined in the appended claims.

Thus the present invention provides a method for assisting diagnosis of rheumatoid arthritis according to claim 1.

The present invention further provides a system suitable for providing information for assisting diagnosis of rheumatoid arthritis according to claim 3.

The present invention also provides a computer program product for enabling a computer to carry out provision of information for assisting diagnosis of arthritis according to claim 5.

According to the present invention, information for distinguishing rheumatoid arthritis from other autoimmune diseases associated with joint abnormality can be obtained, and thus diagnosis of rheumatoid arthritis can be assisted.

### BRIEF DESCRIPTION OF THE DRAWINGS

Fig. 1A shows a scatter diagram illustrating the MIP-1α concentration in sera from a group of patients with anti-CCP antibody-negative rheumatoid arthritis (ACPA-RA group) and a group of patients with arthritis related to autoimmune diseases (ArAD group), and an ROC curve;
Fig. 1B shows a scatter diagram illustrating the MIP-3α concentration in sera from the ACPA-RA group and the ArAD group, and an ROC curve;
Fig. 1C shows a scatter diagram illustrating the BLC concentration in sera from the ACPA-RA group and the ArAD group, and an ROC curve;
Fig. 1D shows a scatter diagram illustrating the Eotaxin concentration in sera from the ACPA-RA group and the ArAD group, and an ROC curve;
Fig. 1E shows a scatter diagram illustrating the MIG concentration in sera from the ACPA-RA group and the ArAD group, and an ROC curve;
Fig. 1F shows a scatter diagram illustrating the CTACK concentration in sera from the ACPA-RA group and the ArAD group, and an ROC curve;
Fig. 1G shows a scatter diagram illustrating the IP-10 concentration in sera from the ACPA-RA group and the ArAD group, and an ROC curve;
Fig. 1H shows a scatter diagram illustrating the TNF-α concentration in sera from the ACPA-RA group and the ArAD group, and an ROC curve;
Fig. 1I shows a scatter diagram illustrating the IL-6 concentration in sera from the ACPA-RA group and the ArAD group, and an ROC curve;
Fig. 1J shows a scatter diagram illustrating the IL-17A concentration in sera from the ACPA-RA group and the ArAD group, and an ROC curve;
Fig. 2A shows a scatter diagram illustrating the MIP-1α concentration in sera from a group of patients with anti-CCP antibody-positive rheumatoid arthritis (ACPA+RA group) and the ArAD group, and an ROC curve;
Fig. 2B shows a scatter diagram illustrating the MIP-3α concentration in sera from the ACPA+RA group and the ArAD group, and an ROC curve;
Fig. 3 is a schematic diagram illustrating an example of a determination device for providing information for assisting diagnosis of rheumatoid arthritis;
Fig. 4 is a block diagram illustrating a functional configuration of the determination device shown in Fig. 3;
Fig. 5 is a block diagram illustrating a hardware configuration of the determination device shown in Fig. 3;
Fig. 6A is a flow chart for determination in order to provide information for assisting diagnosis of rheumatoid arthritis using the determination device shown in Fig. 3; and
Fig. 6B is a flow chart for determination in order to provide information for assisting diagnosis of rheumatoid arthritis using the determination device shown in Fig. 3.

### MODES FOR CARRYING OUT THE INVENTION

In the method for assisting diagnosis of rheumatoid arthritis of the present invention (hereinafter also merely referred to as "method"), at least one chemokine selected from MIP-1α and MIP-3α in a blood sample derived from a subject exhibiting joint abnormality with suspected autoimmune disease is measured.

In the present invention, the subject exhibiting joint abnormality with suspected autoimmune disease may be a subject exhibiting joint abnormality which may be manifested in conjunction with autoimmune diseases such as joint swelling, pain, inflammation, ankylosis, degeneration and destruction. However, the subject according to the present invention does not include a patient with osteoarthritis because osteoarthritis can be easily differentiated from rheumatoid arthritis by current examinations such as radiography. Therefore the method of the present invention is particularly suitable for a subject for whom differentiation between rheumatoid arthritis and other autoimmune diseases associated with joint abnormality is difficult. However, the method of the present invention is not limited to the embodiment in which the subject is the one having a negative test result for anti-CCP antibody because MIP-1α and MIP-3α used as markers for rheumatoid arthritis in the present invention can distinguish anti-CCP antibody-positive rheumatoid arthritis from other autoimmune diseases associated with joint abnormality with high accuracy. Examples of other autoimmune diseases associated with joint abnormality (namely autoimmune diseases other than rheumatoid arthritis) include Behcet's disease, ankylosing spondylitis, psoriatic arthritis, polymyalgia rheumatica and adult-onset Still's disease.

A blood sample used in the present invention includes blood (whole blood), serum and plasma obtained from a subject. Among these, serum is particularly preferable. The blood sample may be a diluted solution obtained by diluting blood, serum or plasma of a subject with a suitable aqueous medium. The aqueous medium is not particularly limited as far as it does not interfere with measurement of the chemokine and includes, for example, water, saline, phosphate-buffered saline (PBS) and the like.

The chemokine measured in the present invention is at least one selected from MIP-1α and MIP-3α. MIP-1α is also referred to as CCL3 (C-C Chemokine Ligand 3) and is known as a chemoattractant of macrophages, monocytes and dendritic cells. MIP-3α is also referred to as CCL20 (C-C Chemokine Ligand 20) and is known as a chemoattractant of dendritic cells and CD45RO+ memory T cells. Amino acid sequences *per se* of MIP-1α and MIP-3α are known in the art and can be retrieved from well-known databases such as the database (http://www.ncbi.nlm.nih.gov/) provided by The National Center for Biotechnology Information (NCBI). In the database provided by the NCBI, the amino acid sequence of MIP-1α is registered under the accession number NM_002983 and the amino acid sequence of MIP-3α is registered under the accession numbers NM_004591 and NM_001130046. These accession numbers are up to date as of the filing date of the present application.

The chemokine may be measured by any method that can provide a value or an index reflecting the amount of MIP-1α and MIP-3α in a blood sample without particular limitation and that is appropriately selected from measurement methods well known in the art. In the present invention, a measurement method that can provide the concentration of MIP-1α and MIP-3α in a blood sample is particularly preferred. Such a measurement method is suitably a measurement method utilizing antigen-antibody reaction including, for example, ELISA, immunoprecipitation, western blotting, affinity chromatography and the like. Among these, ELISA is particularly preferred. When ELISA is used, the concentration of the chemokine can be obtained as follows: that is, a serial dilution of chemokine to be measured having known concentrations is first prepared and a calibration curve is generated based on the results of measurement of the serial dilution. A sample from a subject is then subjected to measurement and the concentration is obtained by applying the measured value to the calibration curve.

An antibody used for measurement of the chemokine is not particularly limited as far as it can specifically bind to the relevant chemokine, i.e. to MIP-1α or MIP-3α. The antibody may be a monoclonal or polyclonal antibody. A fragment (Fab, F(ab')₂, etc.) of the antibody may be used. Methods for preparation of the antibody and fragments thereof are well known in the art.

The chemokine may be measured with commercial antibodies or kits for cytokine (or chemokine) measurement. Specific examples of measurement kits include Duoset ELISA measurement kits (available from R&D systems) and the like. Instead of the antibody, an aptamer which can specifically bind to each of MIP-1α and MIP-3α may be used. Production methods of such an aptamer are well known. For example, an aptamer which can specifically bind to a target protein can be selected by subjecting nucleic acid libraries having random base sequences to the SELEX (Systematic Evolution of Ligands by Exponential enrichment) method.

An antibody and aptamer used for measurement of the chemokine may be labelled with a labelling substance well known in the art. Examples of the labelling substance include radioisotopes such as ³²P, ³⁵S, ³H and ¹²⁵I; fluorescence substances such as fluorescein and Alexa Fluor®; enzymes such as alkaline phosphatase and horseradish peroxidase; biotin, avidin, streptavidin and the like.

In the method of the present invention, information for distinguishing rheumatoid arthritis from other autoimmune diseases associated with joint abnormality is then obtained based on the result of the measurement of the chemokine, as defined in the appended claim. Examples of the information include information indicating that the subject is likely to have rheumatoid arthritis and information indicating that the subject is unlikely to have rheumatoid arthritis (namely the subject is likely to have other autoimmune diseases associated with joint abnormality).

In the present invention, the information for distinguishing rheumatoid arthritis from other autoimmune diseases associated with joint abnormality is obtained by comparing the above concentration of the chemokine with a predetermined threshold. The threshold is established for each of MIP-1α and MIP-3α. The threshold for each chemokine is not particularly limited and can be empirically extablished from accumulated data on various blood samples. Alternatively the predetermined threshold may be established as follows: that is, blood samples from patients with rheumatoid arthritis (particularly anti-CCP antibody-negative patients) and blood samples from patients with other autoimmune diseases associated with joint abnormality are first subjected to measurement to obtain the concentration of MIP-1α and MIP-3α. The values of the concentration obtained are then statistically analyzed by, for example, receiver operating characteristic (ROC) analysis and a value is determined that can distinguish patients with rheumatoid arthritis from patients with other autoimmune diseases associated with joint abnormality with high accuracy. The value is established as the threshold.

If the concentration of the chemokine is at or higher than the predetermined threshold as a result of comparison of the concentration of the chemokine with the threshold, information indicating that the subject is likely to have rheumatoid arthritis can be obtained. To the contrary, if the concentration of the chemokine is less than the predetermined threshold, information indicating that the subject is unlikely to have rheumatoid arthritis can be obtained. Allowing provision of such information, the method of the present invention can assist diagnosis of rheumatoid arthritis on a subject.

The method of the present invention is combined with a test of anti-CCP antibody. The method for assisting diagnosis of rheumatoid arthritis includes a step of measuring anti-CCP antibody in a blood sample from a subject which is hereinafter described.

In the embodiment, at least one chemokine selected from MIP-1α and MIP-3α in a blood sample derived from a subject exhibiting joint abnormality with suspected autoimmune disease is measured. The subject, blood sample and measurement of the chemokine are as described above.

In the embodiment, anti-CCP antibody in the blood sample is further measured. The order of measurement of the chemokine and measurement of anti-CCP antibody is not particularly limited. Thus the chemokine and anti-CCP antibody may be measured in any order or may be measured simultaneously. Anti-CCP antibody may be measured by any method that can provide a value or an index reflecting the amount of anti-CCP antibody in a blood sample without particular limitation. Anti-CCP antibody is particularly preferably measured by a method that can provide a unit of activity per unit volume (hereinafter also referred to as "titre") of anti-CCP antibody. It is the mainstream of current anti-CCP antibody tests to measure the titre (units/mL) of anti-CCP antibody in serum or plasma by enzyme immunoassay (particularly ELISA) using a synthetic CCP as a molecule that can specifically bind to anti-CCP antibody, and it is desirable to follow this in the method of the present invention.

The anti-CCP antibody may be measured with commercial anti-CCP antibody measurement reagents or kits. The reagents and kits are preferably those approved for clinical examinations or *in vitro* diagnosis including, for example, ECLusys reagent Anti-CCP (available from Roche Diagnostics).

In the embodiment, based on the results of the measurement of the chemokine and the measurement of the anti-CCP antibody, information for distinguishing rheumatoid arthritis from other autoimmune diseases associated with joint abnormality is obtained. Examples of the information include information indicating that the subject is likely to have rheumatoid arthritis and information indicating that the subject is unlikely to have rheumatoid arthritis (namely the subject is likely to have other autoimmune diseases associated with joint abnormality).

In the embodiment, it is preferable that information for distinguishing rheumatoid arthritis from other autoimmune diseases associated with joint abnormality is obtained by comparing the concentration of the chemokine with a first threshold and comparing the titre of anti-CCP antibody with a second threshold. The first threshold is established for each of MIP-1α and MIP-3α. The first threshold can be obtained in the same manner as described above. The second threshold is desirably the same standard value (4.5 units/mL) as current anti-CCP antibody tests.

If, as a result of the comparisons, the concentration of the chemokine is at or higher than the first threshold and the titre of anti-CCP antibody is at or higher than the second threshold, or the concentration of the chemokine is at or higher than the first threshold and the tire of anti-CCP antibody is less than the second threshold, or the concentration of the chemokine is less than the first threshold and the titre of anti-CCP antibody is at or higher than the second threshold, then information indicating that the subject is likely to have rheumatoid arthritis can be obtained. To the contrary, if the concentration of the chemokine is less than the first threshold and the titre of anti-CCP antibody is less than the second threshold, information indicating that the subject is unlikely to have rheumatoid arthritis (namely the subject is likely to have other autoimmune diseases associated with joint abnormality) can be obtained. As described above, in the embodiment, based on the result of anti-CCP antibody measurement and the results of MIP-1α and MIP-3α measurements, information on a subject for distinguishing rheumatoid arthritis from other autoimmune diseases associated with joint abnormality can be obtained.

The present invention also encompasses a system suitable for provision of information for assisting diagnosis of rheumatoid arthritis. An example of the system includes the one described hereinbelow.

A system suitable for providing information for assisting diagnosis of rheumatoid arthritis according to claim 3 is also foreseen.

The present invention also encompasses a computer program product for enabling a computer to carry out provision of information for assisting diagnosis of rheumatoid arthritis according to claim 5.

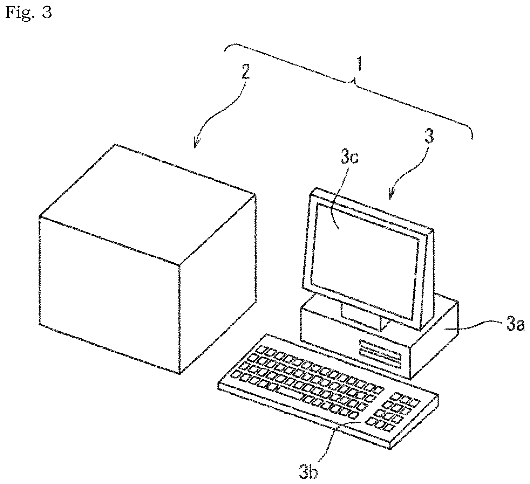

Embodiments of a suitable device for carrying out the method of the present invention are hereinafter described with reference to the drawings. Fig. 3 is a schematic diagram illustrating an example of a determination device for providing information for assisting diagnosis of rheumatoid arthritis. A determination device 1 shown in Fig. 3 includes a measurement device 2 and a computer system 3 connected to the measurement device 2.

In the embodiment, the measurement device 2 is a plate reader for detecting signals generated in ELISA using labelled antibodies. In the embodiment, the signal is optical information such as chemiluminescence signals or fluorescence signals. When an ELISA plate on which antigen-antibody reaction has been carried out is mounted on the measurement device 2, the measurement device 2 obtains optical information based on labelled antibodies specifically bound to the respective MIP-1α and MIP-3α and sends the obtained optical information to the computer system 3. The optical information obtained on the measurement device 2 includes information on specimens having known concentrations in order to calculate the concentration. When anti-CCP antibody is also measured, the measurement device 2 obtains optical information based on a labelled CCP bound to anti-CCP antibody and sends the obtained optical information to the computer system 3.

The plate reader is not particularly limited as far as it can detect signals based on labelling substances used with antibodies specifically binding to the respective MIP-1α and MIP-3α and CCP. As signals vary according to labelling substances, the plate reader can be appropriately selected according to the type of a labelling substance. When, for example, a labelling substance is a radioactive substance, the measurement device 2 used is a plate reader that can detect a radiation generated from the radioactive substance.

The computer system 3 includes a computer main body 3a, an input device 3b and display 3c for displaying sample information, determination results and the like. The computer system 3 receives optical information from the measurement device 2. A processor in the computer system 3 executes, based on the optical information, a programme for determining whether the subject is likely to have rheumatoid arthritis or not.

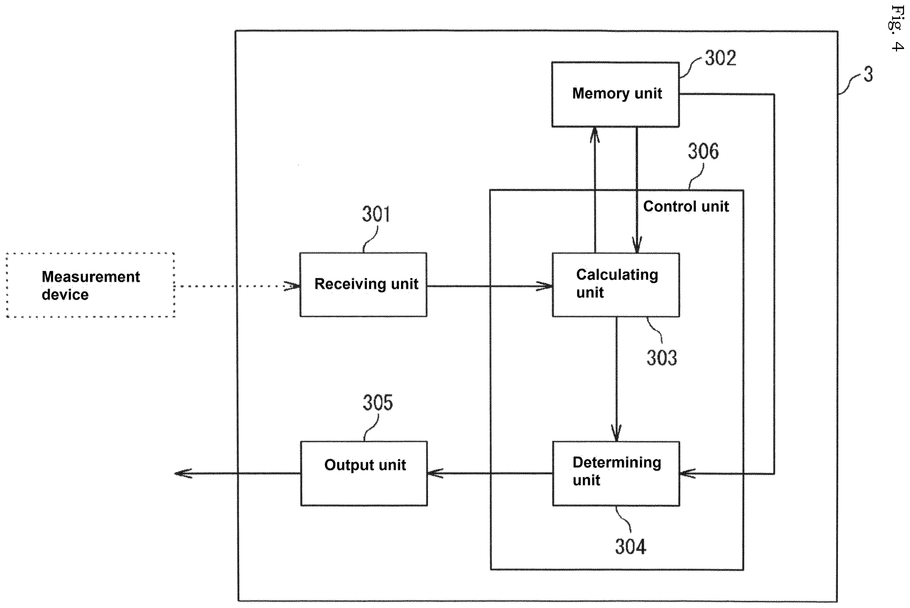

Fig. 4 is a block diagram illustrating a functional configuration of the determination device shown in Fig. 3. As shown in Fig. 4, the computer system 3 includes a receiving unit 301, a memory unit 302, a calculating unit 303, a determining unit 304 and an output unit 305. The receiving unit 301 is communicably connected to the measurement device 2 through a network. The calculating unit 303 and the determining unit 304 are included in a control unit 306.

The receiving unit 301 obtains information from the measurement device 2. The memory unit 302 stores a threshold necessary for determination, a formula for calculation of the concentration of the chemokine and a formula for calculation of the titre of anti-CCP antibody. The calculating unit 303 calculates the concentration of the chemokine according to the formula for calculation of the concentration stored in the memory unit 302 from information obtained at the receiving unit 301. The determining unit 304 determines, based on the concentration calculated at the calculating unit 303 and the threshold stored in the memory unit 302, whether the subject is likely to have rheumatoid arthritis or not. The output unit 305 outputs the determination result from the determining unit 304.

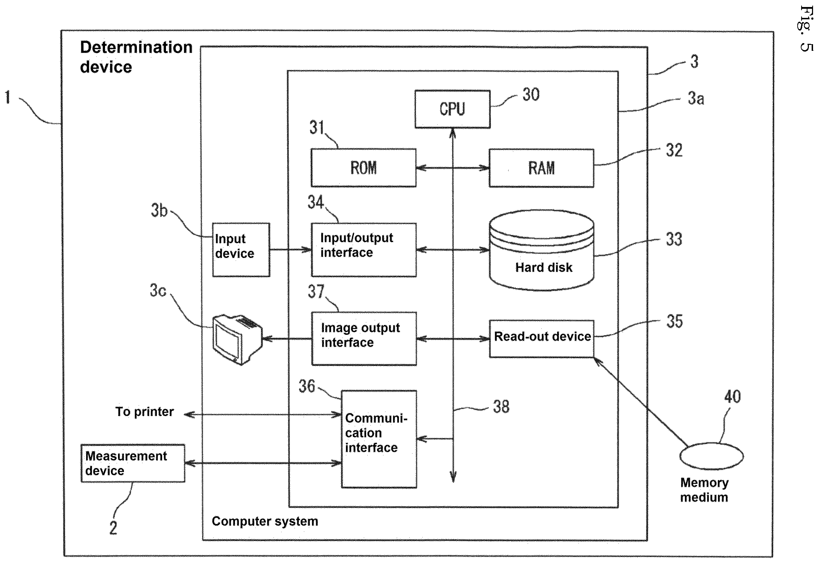

Fig. 5 is a block diagram illustrating a hardware configuration of the determination device shown in Fig. 3. As shown in Fig. 5, the computer main body 3a includes a CPU (Central Processing Unit) 30, ROM (Read Only Memory) 31, RAM (Random Access Memory) 32, a hard disk 33, an input/output interface 34, a read-out device 35, a communication interface 36 and an image output interface 37. The CPU 30, ROM 31, RAM 32, the hard disk 33, the input/output interface 34, the read-out device 35, the communication interface 36 and the image output interface 37 are connected via a bus 38 so as to be able to communicate with each other.

The CPU 30 can execute a computer program stored in ROM 31 and a computer program loaded with RAM 32. When the CPU 30 executes the application program, the functional blocks described above are thereby executed. Accordingly the computer system serves as a terminal that is a determination device for determining whether a subject is likely to have rheumatoid arthritis or not.

ROM 31 is made up with mask ROM, PROM, EPROM, EEPROM or the like. ROM 31 stores the computer program executed by the CPU 30 and data used for the execution.

RAM 32 is made up with SRAM, DRAM or the like. RAM 32 is used for readout of the computer programs stored in ROM 31 and the hard disk 33. RAM 32 is also utilized as a work area when the CPU 30 executes these computer programs.

An operating system to be executed by the CPU 30, computer programs such as application programs (a computer program for determining whether a subject is likely to have rheumatoid arthritis or not) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE1394 and RS-232C, a parallel interface such as SCSI, IDE and IEEE1284 and an analogue interface formed by a D/A converter or an A/D converter. The input/output interface 34 is connected to the input device 3b such as a keyboard and a mouse. A user can input data into the computer main body 3a by means of the input device 3b.

The communication interface 36 is, for example, an Ethernet® interface. The computer system 3 can send printing data to a printer via the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

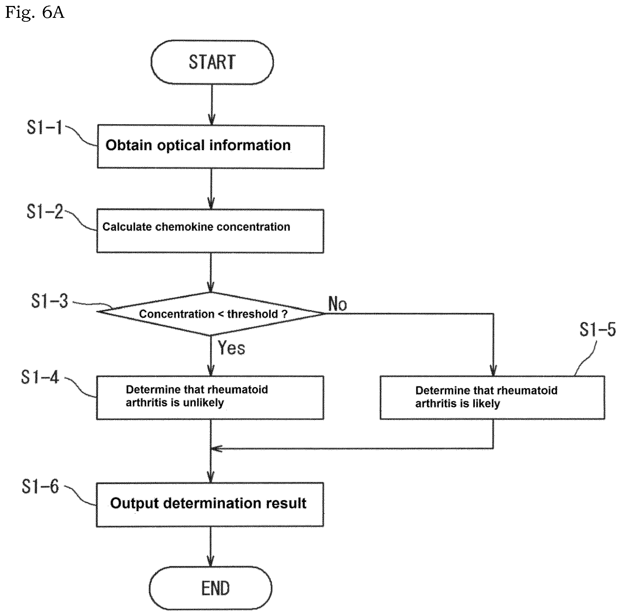

Hereinafter is described the process operations carried out by the determination device 1 for determining whether the subject is likely to have rheumatoid arthritis or not. Fig. 6A is a flow chart for determination on whether the subject is likely to have rheumatoid arthritis or not by using the determination device shown in Fig. 3. An embodiment described herein is a determination based on the concentration of the chemokine obtained from optical information based on labelled antibodies specifically bound to the respective MIP-1α and MIP-3α. However, the present invention is not limited to the embodiment.

In the step S1-1, the receiving unit 301 in the determination device 1 receives from the measurement device 2 optical information. In the next step S1-2, the calculating unit 303 calculates from the optical information received at the receiving unit 301 a concentration of the chemokine according to the formula for calculation of the concentration stored in the memory unit 302.

In the step S1-3 thereafter, the determining unit 304 determines, using the concentration calculated at the calculating unit 303 and the threshold stored in the memory unit 302, whether a subject is likely to have rheumatoid arthritis or not. If the concentration of the chemokine is lower than the threshold, the process proceeds to the step S1-4 and the determining unit 304 sends a determination result indicating that the subject is unlikely to have rheumatoid arthritis to the output unit 305. If the concentration of the chemokine is not lower than the threshold (i.e., the concentration of the chemokine is at or higher than the threshold), the process proceeds to the step S1-5 and the determining unit 304 sends a determination result indicating that the subject is likely to have rheumatoid arthritis to the output unit 305.

In the step S1-6, the output unit 305 outputs the determination result, so that the display 3c displays the result and/or the printer prints out the result. Accordingly, the determination device can provide, to a physician or the like, information for assisting diagnosis on a subject whether or not the subject has rheumatoid arthritis.

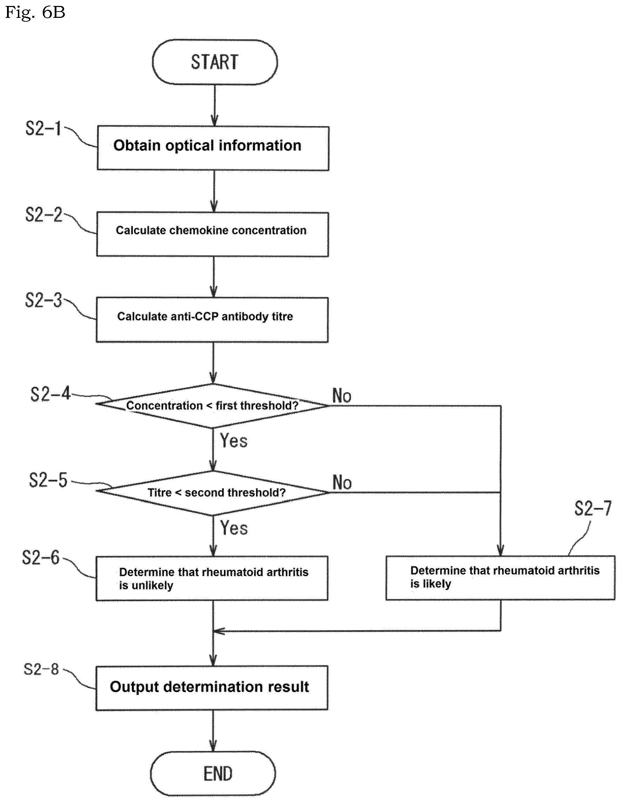

Fig. 6B is a flow chart when anti-CCP antibody is also measured. An embodiment described herein is a determination based on the concentration of the chemokine and the titre of anti-CCP antibody obtained from optical information based on labelled antibodies specifically bound to the respective MIP-1α and MIP-3α and labelled CCP. However, the present invention is not limited to the embodiment.

In the step S2-1, the receiving unit 301 in the determination device 1 receives from the measurement device 2 optical information. In the next step S2-2, the calculating unit 303 calculates from the optical information received at the receiving unit 301 a concentration of the chemokine according to the formula for calculation of the concentration stored in the memory unit 302. In the step S2-3, the titre of anti-CCP antibody is calculated from the optical information on anti-CCP antibody according to the formula for calculation of the titre stored in the memory unit 302.

In the step S2-4 thereafter, the determining unit 304 compares the concentration calculated on the calculating unit 303 and the first threshold stored in the memory unit 302. If the concentration of the chemokine is lower than the first threshold, the process proceeds to the step S2-5 and the determining unit 304 determines, using the titre of anti-CCP antibody calculated on the calculating unit 303 and the second threshold stored in the memory unit 302, whether the subject is likely to have rheumatoid arthritis or not. If the titre of anti-CCP antibody is lower than the second threshold, the process proceeds to the step S2-6 and the determining unit 304 sends a determination result indicating that the subject is unlikely to have rheumatoid arthritis to the output unit 305.

If in the step S2-4 the concentration of the chemokine is not lower than the first threshold (i.e., the concentration of the chemokine is at or higher than the first threshold), the process proceeds to the step S2-7 and the determining unit 304 sends a determination result indicating that the subject is likely to have rheumatoid arthritis to the output unit 305. If in the step S2-5 the titre of anti-CCP antibody is not lower than the second threshold (i.e., the titre of anti-CCP antibody is at or higher than the second threshold), the process proceeds to the step S2-7 and the determining unit 304 sends a determination result indicating that the subject is likely to have rheumatoid arthritis to the output unit 305. In the embodiment, the order of the determination steps, i.e., the step S2-4 and the step S2-5, may be changed.

In the step S2-8, the output unit 305 outputs the determination result, so that the display 3c displays the result and/or the printer prints out the result. Accordingly, the determination device can provide, to a physician or the like, information for assisting diagnosis on a subject whether or not the subject has rheumatoid arthritis.

The present invention is hereinafter more specifically described by way of Examples which do not limit the present invention.

### Examples

Example 1: Comparison of serum marker concentration between rheumatoid arthritis patients and healthy subjects or non-rheumatoid arthritis patients

In the present Example, the serum concentration of various markers was compared in order examine whether MIP-1α and MIP-3α markers can distinguish rheumatoid arthritis patients from healthy subjects and non-rheumatoid arthritis patients. The term "non-rheumatoid arthritis patients" as used herein denotes patients having diseases associated with joint abnormality without rheumatoid arthritis. The similar test was carried out as a control using each of known rheumatoid arthritis markers TNF-α, IL-6 and IL-17A and chemokines BLC, Eotaxin, MIG, CTACK and IP-10.

### 1. Blood samples

Blood samples derived from rheumatoid arthritis patients were sera from rheumatoid arthritis patients (n = 104). The sera of rheumatoid arthritis patients were obtained from two healthcare centres in Japan (hereinafter one of two healthcare centres is referred to as "centre A" and the other is referred to as "centre B"). Blood samples derived from non-rheumatoid arthritis patients used included sera from patients with Behcet's disease (n = 6), patients with ankylosing spondylitis (n = 2), patients with psoriatic arthritis (n = 3), patients with polymyalgia rheumatica (n = 9), patients with adult-onset Still's disease (n = 8) and patients with osteoarthritis (n = 5). All sera of the non-rheumatoid arthritis patients were obtained from the centre B. Blood samples derived from healthy subjects used included sera from healthy volunteers (n = 43).

The rheumatoid arthritis patients from each centre were divided into two groups by random selection using StatFlex V6.0 (available from Artec K.K.). Specifically, the rheumatoid arthritis patients from the centre A were divided into RA(A1) group and RA(A2) group and the rheumatoid arthritis patients from the centre B were divided into RA(B1) group and RA(B2) group. The RA(A2) group and the RA(B2) group were combined to obtain RA(C1) group. For convenience sake, the group of the non-rheumatoid arthritis patients is referred to as Non-RA group and the group of the healthy subjects is referred to as HC group in Example 1. The number of subjects in each group is shown in Table 1.

**Table 1**

| Disease | Group | Number of subjects |
|---|---|---|
| Healthy subjects | HC | 43 |
| Behcet's disease | Non-RA | 33 |
| Ankylosing spondylitis | | |
| Psoriatic arthritis | | |
| Polymyalgia rheumatica | | |
| Adult-onset Still's disease | | |
| Osteoarthritis | | |
| Rheumatoid arthritis | RA(A1) | 29 |
| | RA(B1) | 22 |
| | RA(C1) | 53 |

### 2. Measurement of markers by ELISA

The markers in the serum samples were measured by ELISA as described hereinbelow.

### (2-1) Measurement of MIP-1α

MIP-1α was measured with an ELISA measurement kit Duoset (DY270) (available from R&D systems). Specifically, measurement was carried out as follows: to each well of a 384-well ELISA plate, 50 µL of an antibody diluted solution (0.4 µg/mL of a solid phase antibody, 0.05% NaN₃, PBS pH 7.4) was added and the antibody was immobilized overnight at 4°C. The plate was washed three times with a washing buffer (0.05% Tween 20, 0.05% NaN₃, PBS pH 7.4) and 100 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 50 µL of serum which was previously 4-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 50 µL of a labelled antibody solution (0.2 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 50 µL of an alkaline phosphatase-labelled streptavidin solution (1:1000 diluted Streptavidin-Alkaline Phosphatase [R&D systems AR001], 1% BSA,0.5% Casein, 0.05% NaN₃, PBS pH 7.4) and the reaction was allowed to proceed for 20 minutes. The plate was washed three times with the washing buffer and further washed once with an ALP activation buffer (20 mM Tris, 10 mM MgCl, pH 9.8). To each well of the plate was added 50 µL of a substrate (CDP-Star® Ready-to-use Sapphire II, available from Tropix, Inc.) and the luminescence signal was detected on a chemiluminescence detector (FLUO star OPTIMA BMG, available from BMG LabTech).

### (2-2) Measurement of MIP-3α

MIP-3α was measured with an ELISA measurement kit Duoset (DY210) (available from R&D systems). Specifically, measurement was carried out as follows: to each well of a 96-well ELISA plate, 100 µL of an antibody diluted solution (2 µg/mL of a solid phase antibody, PBS pH 7.4) was added and the antibody was immobilized overnight at 4°C. The plate was washed four times with the washing buffer and 250 µL of a blocking buffer (1% BSA, 0.05% Tween 20, 0.05% NaN₃, PBS pH 7.4) was added to each well followed by blocking at room temperature for 2 hours or more. To each well of the plate was added 100 µL of serum which was previously 4-fold diluted with an ELISA dilution buffer (1% BSA, 0.05% NaN₃, PBS pH7.4) and the reaction was allowed to proceed overnight at 4°C. The plate was washed three times with the washing buffer. To each well of the plate was added 100 µL of a labelled antibody solution (0. 5 µg/mL of a labelled antibody, 1% BSA, 0.05% NaN₃, PBS pH 7.4) and the reaction was allowed to proceed at room temperature for 2 hours. The plate was washed three times with the washing buffer. To each well of the plate was added 100 µL of an alkaline phosphatase-labelled streptavidin solution (1:1000 diluted Streptavidin-Alkaline Phosphatase, 1% BSA, 0.05% NaN₃, PBS pH 7.4) and the reaction was allowed to proceed for 20 minutes. The plate was washed three times with the washing buffer and further washed once with the ALP activation buffer. To each well of the plate was added 100 µL of a substrate (CDP-Star® Ready-to-use Sapphire II) and the luminescence signal was detected on a chemiluminescence detector (FLUO star OPTIMA BMG).

### (2-3) Measurement of TNF-α

TNF-α was measured with an ELISA measurement kit Duoset (DY360) (available from R&D systems). Specifically, measurement was carried out as follows: to each well of a 384-well ELISA plate, 50 µL of an antibody diluted solution (4 µg/mL of a solid phase antibody, PBS pH 7.4) was added and the antibody was immobilized overnight at 4°C. The plate was washed three times with the washing buffer and 100 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 50 µL of serum which was previously 100-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 50 µL of a labelled antibody solution (0.35 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 50 µL of an alkaline phosphatase-labelled streptavidin solution (1:1000 diluted Streptavidin-Alkaline Phosphatase, 1% BSA, 0.5% Casein, 0.05% NaN₃, PBS pH 7.4) and the reaction was allowed to proceed for 20 minutes. The plate was washed three times with the washing buffer and further washed once with the ALP activation buffer. To each well of the plate was added 50 µL of a substrate (CDP-Star® Ready-to-use Sapphire II) and the luminescence signal was detected on a chemiluminescence detector (FLUO star OPTIMA BMG).

### (2-4) Measurement of IL-6

IL-6 was measured with an ELISA measurement kit Duoset (DY206) (available from R&D systems). Specifically, measurement was carried out as follows: to each well of a 96-well ELISA plate, 100 µL of an antibody diluted solution (2 µg/mL of a solid phase antibody, PBS pH 7.4) was added and the antibody was immobilized overnight at 4°C. The plate was washed three times with the washing buffer and 250 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 100 µL of serum which was previously 4-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 100 µL of a labelled antibody solution (0.2 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 100 µL of an alkaline phosphatase-labelled streptavidin solution (1:1000 diluted Streptavidin-Alkaline Phosphatase, 1% BSA, 0.5% Casein, 0.05% NaN₃, PBS pH 7.4) and the reaction was allowed to proceed for 20 minutes. The plate was washed three times with the washing buffer and further washed once with the ALP activation buffer. To each well of the plate was added 100 µL of a substrate (CDP-Star® Ready-to-use Sapphire II) and the luminescence signal was detected on a chemiluminescence detector (FLUO star OPTIMA BMG).

### (2-5) Measurement of IL-17A

IL-17A was measured with a solid phase antibody of 64CAP17eBio (available from eBioscience) and a labelled antibody of 64DEC17eBio (available from eBioscience). Specifically, measurement was carried out as follows: to each well of a 384-well ELISA plate, 50 µL of an antibody diluted solution (1 µg/mL of the solid phase antobidy, PBS pH 7.4) was added and the antibody was immobilized overnight at 4°C. The plate was washed three times with the washing buffer and 100 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 50 µL of serum which was previously 100-fold diluted with an ELISA dilution buffer (1% BSA,0.5% Casein, 0.05% NaN₃, PBS pH 7.4) and the reaction was allowed to proceed overnight at 4°C. The plate was washed three times with the washing buffer. To each well of the plate was added 50 µL of a labelled antibody solution (0.5 µg/mL of the labelled antibody, 1% BSA, 0.5% Casein, 0.05% NaN₃, PBS pH7.4) and the reaction was allowed to proceed at room temperature for 2 hours. The plate was washed three times with the washing buffer. To each well of the plate was added 50 µL of an alkaline phosphatase-labelled streptavidin solution (1:1000 diluted Streptavidin-Alkaline Phosphatase, 1% BSA, 0.5% Casein, 0.05% NaN₃, PBS pH 7.4) and the reaction was allowed to proceed for 20 minutes. The plate was washed three times with the washing buffer and further washed once with the ALP activation buffer. To each well of the plate was added 50 µL of a substrate (CDP-Star® Ready-to-use Sapphire II) and the luminescence signal was detected on a chemiluminescence detector (FLUO star OPTIMA BMG).

### (2-6) Measurement of BLC

BLC was measured with an ELISA measurement kit Duoset (DY801) (available from R&D systems). Specifically, measurement was carried out as follows: to each well of a 96-well ELISA plate, 100 µL of an antibody diluted solution (2 µg/mL of a solid phase antibody, PBS pH 7.4) was added and the antibody was immobilized overnight at 4°C. The plate was washed three times with the washing buffer and 250 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 100 µL of serum which was previously 4-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 100 µL of a labelled antibody solution (0.1 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 100 µL of an alkaline phosphatase-labelled streptavidin solution (1:1000 diluted Streptavidin-Alkaline Phosphatase, 1% BSA, 0.5% Casein, 0.05% NaN₃, PBS pH 7.4) and the reaction was allowed to proceed for 20 minutes. The plate was washed three times with the washing buffer and further washed once with the ALP activation buffer. To each well of the plate was added 100 µL of a substrate (CDP-Star® Ready-to-use Sapphire II) and the luminescence signal was detected on a chemiluminescence detector (FLUO star OPTIMA BMG).

### (2-7) Measurement of Eotaxin

Eotaxin was measured with an ELISA measurement kit Duoset (DY420) (available from R&D systems). Specifically, measurement was carried out as follows: to each well of a 96-well ELISA plate, 100 µL of an antibody diluted solution (2 µg/mL of a solid phase antibody, PBS pH 7.4) was added and the antibody was immobilized overnight at 4°C. The plate was washed three times with the washing buffer and 250 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 100 µL of serum which was previously 4-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 100 µL of a labelled antibody solution (0.1 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 100 µL of an alkaline phosphatase-labelled streptavidin solution (1:1000 diluted Streptavidin-Alkaline Phosphatase, 1% BSA, 0.5% Casein, 0.05% NaN₃, PBS pH 7.4) and the reaction was allowed to proceed for 20 minutes. The plate was washed three times with the washing buffer and further washed once with the ALP activation buffer. To each well of the plate was added 100 µL of a substrate (CDP-Star® Ready-to-use Sapphire II) and the luminescence signal was detected on a chemiluminescence detector (FLUO star OPTIMA BMG).

### (2-8) Measurement of MIG

MIG was measured with an ELISA measurement kit Duoset (DY392) (available from R&D systems). Specifically, measurement was carried out as follows: to each well of a 96-well ELISA plate, 100 µL of an antibody diluted solution (1 µg/mL of a solid phase antibody, PBS pH 7.4) was added and the antibody was immobilized overnight at 4°C. The plate was washed three times with the washing buffer and 250 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 100 µL of serum which was previously 4-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 100 µL of a labelled antibody solution (0.2 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 100 µL of an alkaline phosphatase-labelled streptavidin solution (1:1000 diluted Streptavidin-Alkaline Phosphatase, 1% BSA, 0.05% NaN₃, PBS pH 7.4) and the reaction was allowed to proceed for 20 minutes. The plate was washed three times with the washing buffer and further washed once with the ALP activation buffer. To each well of the plate was added 100 µL of a substrate (CDP-Star® Ready-to-use Sapphire II) and the luminescence signal was detected on a chemiluminescence detector (FLUO star OPTIMA BMG).

### (2-9) Measurement of CTACK

CTACK was measured with an ELISA measurement kit Duoset (DY376) (available from R&D systems). Specifically, measurement was carried out as follows: to each well of a 96-well ELISA plate, 100 µL of an antibody diluted solution (4 µg/mL of a solid phase antibody, PBS pH 7.4) was added and the antibody was immobilized overnight at 4°C. The plate was washed three times with the washing buffer and 250 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 100 µL of serum which was previously 4-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 100 µL of a labelled antibody solution (0.075 µg/mL of a labelled antibody, 1% BSA, 0.5% Casein, 0.05% NaN₃, PBS pH 7.4) and the reaction was allowed to proceed at room temperature for 2 hours. The plate was washed three times with the washing buffer. To each well of the plate was added 100 µL of an alkaline phosphatase-labelled streptavidin solution (1:1000 diluted Streptavidin-Alkaline Phosphatase, 1% BSA, 0.05% NaN₃, PBS pH 7.4) and the reaction was allowed to proceed for 20 minutes. The plate was washed three times with the washing buffer and further washed once with the ALP activation buffer. To each well of the plate was added 100 µL of a substrate (CDP-Star® Ready-to-use Sapphire II) and the luminescence signal was detected on a chemiluminescence detector (FLUO star OPTIMA BMG).

### (2-10) Measurement of IP-10

IP-10 was measured with an ELISA measurement kit Duoset (DY266) (available from R&D systems). Specifically, measurement was carried out as follows: to each well of a 384-well ELISA plate, 50 µL of an antibody diluted solution (2 µg/mL of a solid phase antibody, PBS pH 7.4) was added and the antibody was immobilized overnight at 4°C. The plate was washed three times with the washing buffer and 100 µL of a blocking buffer (1% BSA, 0.5% Casein, 0.05% Tween 20, 0.05% NaN₃, PBS pH 7.4) was added to each well followed by blocking at room temperature for 2 hours or more. To each well of the plate was added 50 µL of serum which was previously 100-fold diluted with an ELISA dilution buffer (1% BSA, 0.5% Casein, 0.05% NaN₃, PBS pH 7.4) and the reaction was allowed to proceed overnight at 4°C. The plate was washed three times with the washing buffer. To each well of the plate was added 50 µL of a labelled antibody solution (0.05 µg/mL of a labelled antibody, 1% BSA, 0.5% Casein, 0.05% NaN₃, PBS pH7.4) and the reaction was allowed to proceed at room temperature for 2 hours. The plate was washed three times with the washing buffer. To each well of the plate was added 50 µL of an alkaline phosphatase-labelled streptavidin solution (1:1000 diluted Streptavidin-Alkaline Phosphatase, 1% BSA, 0.5% Casein, 0.05% NaN₃, PBS pH 7.4) and the reaction was allowed to proceed for 20 minutes. The plate was washed three times with the washing buffer and further washed once with the ALP activation buffer. To each well of the plate was added 50 µL of a substrate (CDP-Star® Ready-to-use Sapphire II) and the luminescence signal was detected on a chemiluminescence detector (FLUO star OPTIMA BMG).

### 3. Obtaining serum marker concentration

For each marker, a calibration curve was generated by the 4-Parameter fit from luminescence signals (RLU) of standards having known concentrations. A luminescence signal (RLU) obtained from the above test measurement was applied to the calibration curve to obtain the serum marker concentration. Generation of the calibration curve and determination of the marker concentration were carried out with an analysis software MARS (available from BMG LabTech).

### 4. Statistical analysis

The serum concentration of each marker was compared between the groups of rheumatoid arthritis patients and the group of healthy subjects or non-rheumatoid arthritis patients. Specifically whether the difference in the serum marker concentration between two groups is significant or not was evaluated by the Mann-Whitney test. The significance test was carried out with StatFlex V6.0 (available from Artec K.K.). The combinations of two groups compared and the test results (p values) are shown in Table 2.

**Table 2**

| | Comparison 1 | Comparison 2 | Comparison 3 | Comparison 4 | Comparison 5 | Comparison 6 |
|---|---|---|---|---|---|---|
| | HC/RA(A1) | HC/RA(B1) | HC/RA(C1) | Non-RA/RA(A1) | Non-RA/RA(B1) | Non-RA/RA(C1) |
| TNF-α | < 0.001 | < 0.013 | < 0.001 | < 0.001 | 0.172 | < 0.001 |
| IL-6 | 0.469 | < 0.001 | < 0.001 | 0.003 | < 0.001 | 0.546 |
| IL-17A | 0.027 | < 0.001 | < 0.001 | 0.532 | 0.029 | 0.168 |
| MIP-1α | < 0.001 | < 0.001 | < 0.001 | < 0.001 | 0.010 | < 0.001 |
| MIP-3α | < 0.001 | 0.048 | 0.005 | < 0.001 | < 0.001 | < 0.001 |
| BLC | < 0.001 | < 0.001 | 0.007 | < 0.001 | < 0.001 | < 0.001 |
| Eotaxin | < 0.001 | < 0.001 | < 0.001 | < 0.001 | 0.036 | < 0.001 |
| MIG | < 0.001 | < 0.001 | < 0.001 | 0.004 | 0.010 | 0.016 |
| CTACK | < 0.001 | < 0.001 | < 0.001 | 0.030 | < 0.001 | 0.022 |
| IP-10 | < 0.001 | < 0.001 | < 0.001 | 0.014 | 0.002 | 0.007 |

It is found from Table 2 that known rheumatoid arthritis markers TNF-α, IL-6 and IL-17A failed to show a statistically significant difference (p < 0.05) in at least one of comparisons 1 to 6. On the other hand, MIP-1α, MIP-3α, BLC, Eotaxin, MIG, CTACK and IP-10 showed a statistically significant difference (p < 0.05) in all comparisons 1 to 6.

Example 2: Evaluation of ability to distinguish patients with anti-CCP antibody-negative rheumatoid arthritis from patients with other autoimmune diseases associated with joint abnormality

In the present Example, the ability of MIP-1α and MIP-3α to distinguish patients with anti-CCP antibody-negative rheumatoid arthritis from patients with other autoimmune diseases associated with joint abnormality was evaluated based on the concentration of markers in various sera. For comparison, similar tests were carried out for TNF-α, IL-6, IL-17A, BLC, Eotaxin, MIG, CTACK and IP-10.

### 1. Blood samples

Blood samples derived from patients with anti-CCP antibody-negative rheumatoid arthritis used were sera having a measured value of anti-CCP antibody of less than 4.5 units/mL among the sera from the rheumatoid arthritis patients used in Example 1 and sera from rheumatoid arthritis patients newly obtained at the centre A and centre B (hereinafter referred to as "RA(C2) group"). In the present Example, the group of patients with anti-CCP antibody-negative rheumatoid arthritis is also referred to as "ACPA-RA group". The ACPA-RA group included 4 subjects from the RA(A1) group, 4 subjects from the RA(B1) group, 17 subjects from the RA(C1) group and 20 subjects from the RA(C2) group.

Blood samples derived from patients with other autoimmune diseases associated with joint abnormality used were sera from Behcet's disease patients, ankylosing spondylitis patients, psoriatic arthritis patients, polymyalgia rheumatica patients and adult-onset Still's disease patients used in Example 1. As these diseases manifest arthritis related to autoimmune diseases, the group of patients with the diseases is hereinafter also referred to as "ArAD group". The number of subjects in each group is shown in Table 3.

**Table 3**

| Disease | Group | Number of subjects |
|---|---|---|
| Behcet's disease | ArAD | 28 |
| Ankylosing spondylitis | | |
| Psoriatic arthritis | | |
| Polymyalgia rheumatica | | |
| Adult-onset Still's disease | | |
| Anti-CCP antibody-negative rheumatoid arthritis | ACPA⁻RA | 45 |

### 2. Measurement of markers by ELISA

The markers in sera from the RA(C2) group were measured by ELISA in the same manner as in Example 1. For the groups other than the RA(C2) group, the data obtained in Example 1 were used.

### 3. Obtaining serum marker concentration

For each marker in the sera from the RA(C2) group, a calibration curve was generated by the 4-Parameter fit from luminescence signals (RLU) of standards having known concentrations. A luminescence signal (RLU) obtained from the above test measurement was applied to the calibration curve to obtain the serum marker concentration. Generation of the calibration curve and determination of the marker concentration were carried out with an analysis software MARS (available from BMG LabTech).

### 4. Statistical analysis

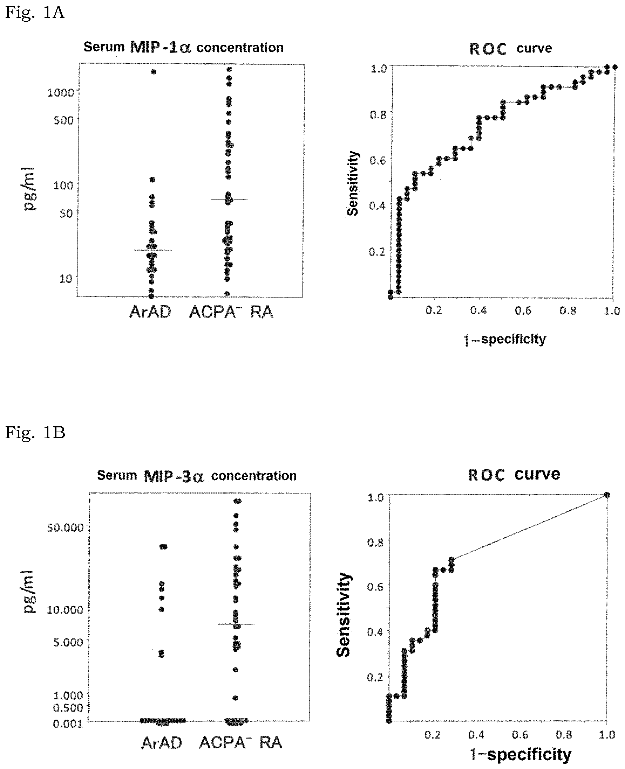

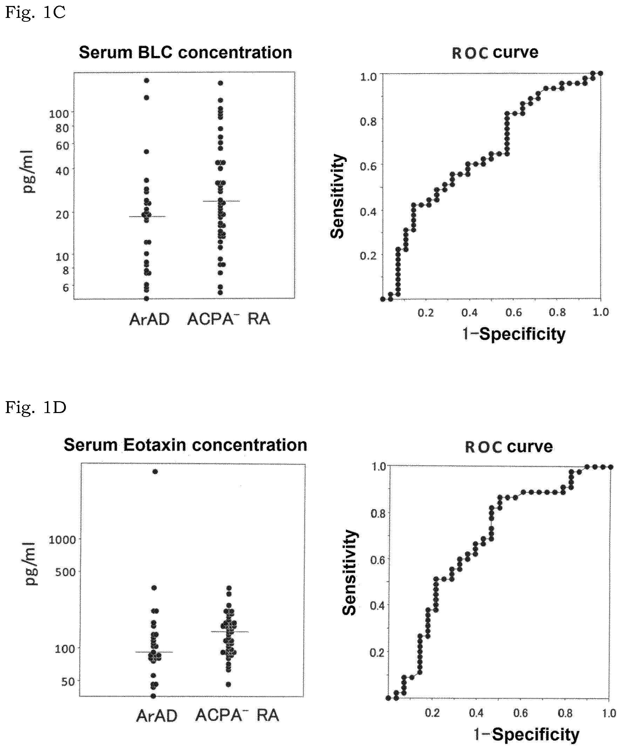

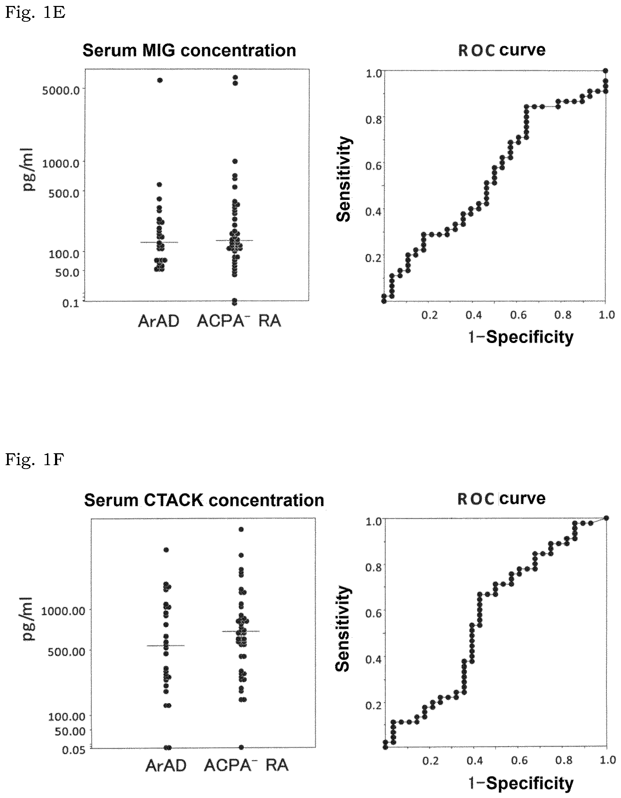

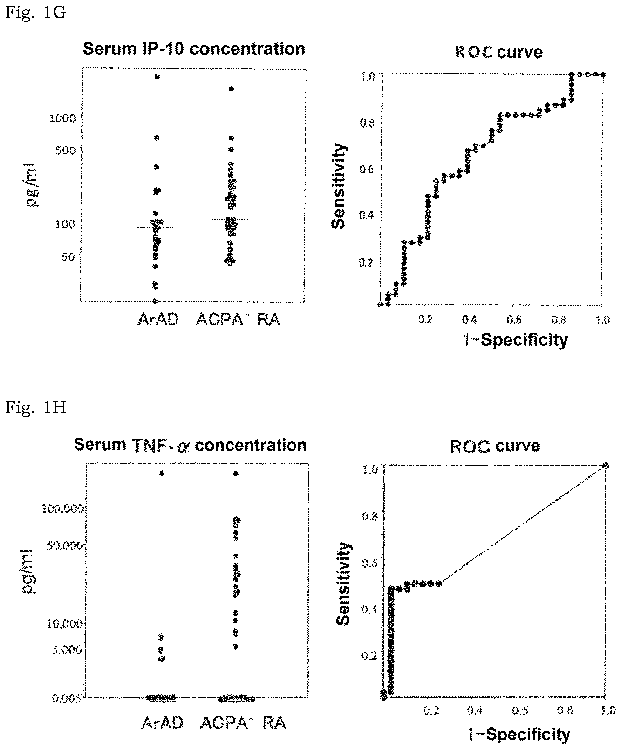

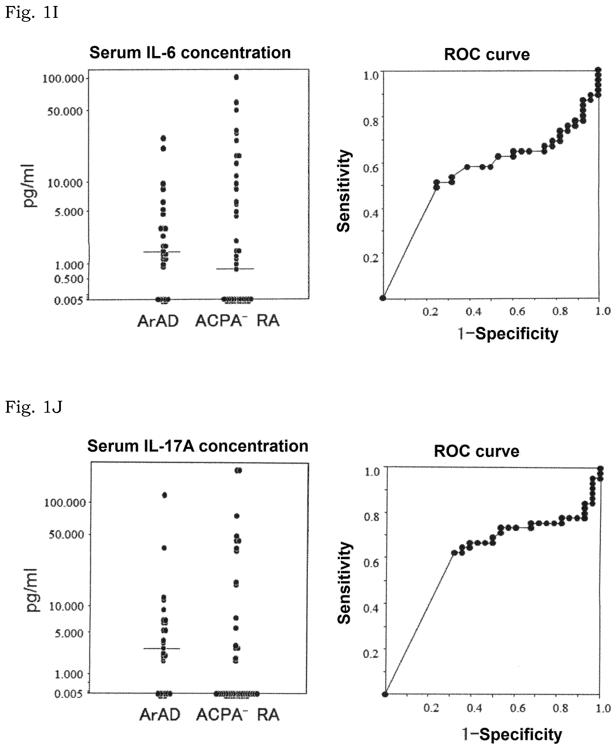

The serum concentration of each marker was compared between the ArAD group and the ACPA-RA group. Specifically whether the difference in the serum marker concentration between two groups is significant or not was evaluated by the Mann-Whitney test. The significance test was carried out with StatFlex V6.0 (available from Artec K.K.). For each marker, an ROC curve was generated from the serum concentration data to obtain the area under the curve (AUC). For each marker, a cut-off between the ArAD group and the ACPA-RA group was established and the sensitivity, specificity, positive predictive value (PPV) and negative predictive value (NPV) were obtained. The cut-off was established as a concentration that provides the maximum sensitivity and specificity. The distribution of the serum concentration, p values and the results of ROC analysis of the respective markers are shown in Fig. 1 and Table 4.

**Table 4**

| Marker | p value | AUC | Cut-off (pg/mL) | Sensitivity (%) | Specificity (%) | PPV (%) | NPV (%) |
|---|---|---|---|---|---|---|---|
| MIP-1α | 0.00051 | 0.743 | 25.2 | 68.9 | 64.3 | 75.6 | 56.3 |
| MIP-3α | 0.00108 | 0.718 | 0.8 | 68.9 | 71.4 | 79.5 | 58.8 |
| BLC | 0.03634 | 0.646 | 19.5 | 60.0 | 60.7 | 71.1 | 48.6 |
| Eotaxin | 0.01495 | 0.670 | 118.6 | 60.0 | 64.3 | 50.0 | 27.0 |
| MIG | 0.54013 | 0.543 | 126.4 | 57.8 | 50.0 | 65.0 | 42.2 |
| CTACK | 0.34638 | 0.566 | 636.7 | 53.3 | 60.7 | 68.6 | 44.7 |
| IP-10 | 0.03949 | 0.644 | 100.6 | 57.8 | 60.7 | 52.8 | 29.7 |
| TNF-α | 0.00866 | 0.662 | 3.7 | 48.6 | 78.6 | 78.6 | 48.9 |
| IL-6 | 0.46748 | 0.549 | 1.3 | 67.9 | 53.4 | 36.7 | 60.5 |
| IL-17A | 0.11804 | 0.602 | 2.0 | 64.3 | 62.2 | 51.5 | 72.5 |

It is found from Fig. 1 and Table 4 that MIP-1α and MIP-3α showed a statistically significant difference (p < 0.05) between the ArAD group and the ACPA-RA group and had an AUC of 0.7 or more. In the field of clinical tests, the AUC of 0.7 or more is generally desirable. On the other hand, BLC, Eotaxin, MIG, CTACK, IP-10, TNF-α, IL-6 and IL-17A either did not show a statistically significant difference (p < 0.05) between the ArAD group and the ACPA-RA group or had an AUC or less than 0.7. Therefore it is demonstrated that MIP-1α and MIP-3α are the markers capable of distinguishing patients with anti-CCP antibody-negative rheumatoid arthritis from patients with other autoimmune diseases associated with joint abnormality.

Example 3: Evaluation of ability to distinguish patients with anti-CCP antibody-positive rheumatoid arthritis and patients with other autoimmune diseases associated with joint abnormality

In the present Example, the ability of MIP-1α and MIP-3α to distinguish patients with anti-CCP antibody-positive rheumatoid arthritis patients from patients with other autoimmune diseases associated with joint abnormality was evaluated based on the concentration of markers in various sera.

### 1. Blood samples

Blood samples derived from patients with anti-CCP antibody-positive rheumatoid arthritis used were sera having a measured value of anti-CCP antibody of 4.5 units/mL or more among sera from the rheumatoid arthritis patients in the RA(A1), RA(B1), RA(C1) and RA(C2) groups described above. In the present Example, the group of patients with anti-CCP antibody-positive rheumatoid arthritis is also referred to as "ACPA⁺RA group". The ACPA+RA group included 25 subjects from the RA(A1) group, 16 subjects from the RA(B1) group, 35 subjects from the RA(C1) group and 28 subjects from the RA(C2) group. Blood samples derived from patients with other autoimmune diseases associated with joint abnormality used were sera from the ArAD group used in Example 2. The number of subjects in each group is shown in Table 5.

**Table 5**

| Disease | Group | Number of subjects |
|---|---|---|
| Behcet's disease | ArAD | 28 |
| Ankylosing spondylitis | | |
| Psoriatic arthritis | | |
| Polymyalgia rheumatica | | |
| Adult-onset Still's disease | | |
| Anti-CCP antibody-positive rheumatoid arthritis | ACPA⁺RA | 104 |

### 2. Measurement of markers by ELISA

The markers in sera from the RA(C2) group were measured by ELISA in the same manner as in Example 1. For the groups other than the RA(C2) group, the data obtained in Example 1 were used.

### 3. Obtaining serum marker concentration

For each marker in the sera from the RA(C2) group, a calibration curve was generated by the 4-Parameter fit from luminescence signals (RLU) of standards having known concentrations. A luminescence signal (RLU) obtained from the above test measurement was applied to the calibration curve to obtain the serum marker concentration. Generation of the calibration curve and determination of the marker concentration were carried out with an analysis software MARS (available from BMG LabTech).

### 4. Statistical analysis

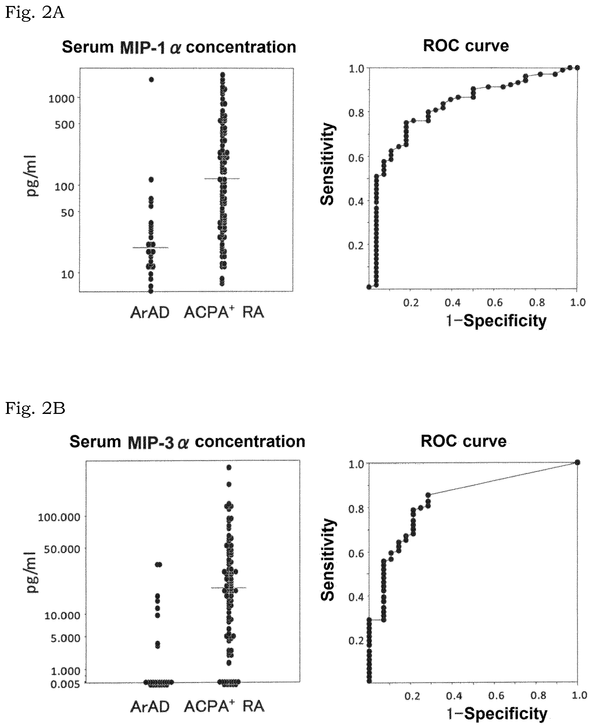

The serum concentration of each marker was compared between the ArAD group and the ACPA+RA group in the same manner as in Example 2. For each marker, an ROC curve was generated from the serum concentration data to obtain the AUC. For each marker, a cut-off between the ArAD group and the ACPA+RA group was established and the sensitivity, specificity, PPV and NPV were obtained. The cut-off was established as a concentration that provides the maximum sensitivity and specificity. The distribution of the serum concentration, p values and the results of ROC analysis of the respective markers are shown in Fig. 2 and Table 6.

**Table 6**

| Marker | p value | AUC | Cut-off (pg/mL) | Sensitivity (%) | Specificity (%) | PPV (%) | NPV (%) |
|---|---|---|---|---|---|---|---|
| MIP-1α | < 0.00001 | 0.821 | 25.2 | 83.7 | 64.3 | 89.7 | 51.4 |
| MIP-3α | < 0.00001 | 0.829 | 0.8 | 85.6 | 71.4 | 91.8 | 57.1 |

It is found from Fig. 2 and Table 6 that MIP-1α and MIP-3α showed a statistically significant difference (p < 0.05) between the ArAD group and the ACPA+RA group and had an AUC of 0.7 or more. Therefore it is demonstrated that MIP-1α and MIP-3α are the markers capable of distinguishing patients with anti-CCP antibody-positive rheumatoid arthritis from patients with other autoimmune diseases associated with joint abnormality. Taking the results of Example 2 into account, it is demonstrated that MIP-1α and MIP-3α can distinguish, regardless of the test results of anti-CCP antibody, rheumatoid arthritis patients from patients with other autoimmune diseases associated with joint abnormality.

## Claims

1. A method for assisting diagnosis of rheumatoid arthritis, comprising steps of:
measuring at least one chemokine and anti-cyclic citrullinated peptide antibody (anti-CCP antibody) in a blood sample derived from a subject exhibiting joint abnormality and obtaining a concentration of the chemokine and a titre of the anti-CCP antibody in the blood sample;
comparing the concentration of the chemokine with a first threshold and comparing the titre of the anti-CCP antibody with a second threshold; and
obtaining information for distinguishing rheumatoid arthritis from other autoimmune diseases associated with joint abnormality, wherein:
if the concentration of the chemokine is at or higher than the first threshold and the titre of the anti-CCP antibody is at or higher than the second threshold, or the concentration of the chemokine is at or higher than first threshold and the titre of the anti-CCP antibody is less than the second threshold, or the concentration of the chemokine is less than the first threshold and the titre of the anti-CCP antibody is at or higher than the second threshold, information indicating that the subject is likely to have rheumatoid arthritis is obtained and
if the concentration of the chemokine is less than the first threshold and the titre of the anti-CCP antibody is less than the second threshold, information indicating that the subject is unlikely to have rheumatoid arthritis is obtained, wherein the chemokine is selected from MIP-1α and MIP-3α.

2. The method according to claim 1, wherein the other autoimmune diseases associated with joint abnormality include at least one selected from Behcet's disease, ankylosing spondylitis, psoriatic arthritis, polymyalgia rheumatica and adult-onset Still's disease.

3. A system suitable for providing information for assisting diagnosis of rheumatoid arthritis, comprising a computer containing a processor and a memory controlled by the processor, wherein the memory includes a computer program for enabling the computer to carry out following steps of:
obtaining a result of measurement of at least one chemokine and anti-cyclic citrullinated peptide antibody (anti-CCP antibody) in a blood sample derived from a subject exhibiting joint abnormality with suspected autoimmune disease;
calculating a concentration of the chemokine and a titre of the anti-CCP antibody in the blood sample; and
comparing the concentration of the chemokine with a first threshold and comparing the titre of the anti-CCP antibody with a second threshold and providing information indicating that the subject is likely to have rheumatoid arthritis if the concentration of the chemokine is at or higher than the first threshold and the titre of the anti-CCP antibody is at or higher than the second threshold, or the concentration of the chemokine is at or higher than first threshold and the titre of the anti-CCP antibody is less than the second threshold, or the concentration of the chemokine is less than the first threshold and the titre of the anti-CCP antibody is at or higher than the second threshold, information indicating that the subject is likely to have rheumatoid arthritis is obtained, or providing information indicating that the subject is unlikely to have rheumatoid arthritis is obtained if the concentration of the chemokine is less than the first threshold and the titre of the anti-CCP antibody is less than the second threshold, whereby chemokine is selected from MIP-1α and MIP-3α.

4. The system according to claim 3, wherein the other autoimmune diseases associated with joint abnormality include at least one selected from Behcet's disease, ankylosing spondylitis, psoriatic arthritis, polymyalgia rheumatica and adult-onset Still's disease.

5. A computer program product for enabling a computer to carry out provision of information for assisting diagnosis of rheumatoid arthritis, comprising a computer readable medium, wherein the medium comprises a computer program for enabling a computer to carry out following steps of:
obtaining a result of measurement of at least one chemokine and anti-cyclic citrullinated peptide antibody (anti-CCP antibody) in a blood sample derived from a subject exhibiting joint abnormality with suspected autoimmune disease;
calculating a concentration of the chemokine and a titre of the anti-CCP antibody in the blood sample; and
comparing the concentration of the chemokine with a first threshold and comparing the titre of the anti-CCP antibody with a second threshold and providing information indicating that the subject is likely to have rheumatoid arthritis if the concentration of the chemokine is at or higher than the first threshold and the titre of the anti-CCP antibody is at or higher than the second threshold, or the concentration of the chemokine is at or higher than first threshold and the titre of the anti-CCP antibody is less than the second threshold, or the concentration of the chemokine is less than the first threshold and the titre of the anti-CCP antibody is at or higher than the second threshold, information indicating that the subject is likely to have rheumatoid arthritis is obtained, or providing information indicating that the subject is unlikely to have rheumatoid arthritis is obtained if the concentration of the chemokine is less than the first threshold and the titre of the anti-CCP antibody is less than the second threshold, whereby the chemokine is selected from MIP-1α and MIP-3α.

## Patentansprüche

1. Verfahren zur Unterstützung der Diagnose von rheumatoider Arthritis, umfassend die Schritte:
Messen von mindestens einem Chemokin und einem Anti-zyklisches-citrulliniertes-Peptid-Antikörper (Anti-CCP-Antikörper) in einer Blutprobe, die von einem Individuum stammt, das eine Gelenkanomalie aufweist, und Erhalten einer Konzentration des Chemokins und eines Titers des Anti-CCP-Antikörpers in der Blutprobe;
Vergleichen der Konzentration des Chemokins mit einem ersten Schwellenwert und Vergleichen des Titers des Anti-CCP-Antikörpers mit einem zweiten Schwellenwert; und
Erhalten einer Information zur Unterscheidung von rheumatoider Arthritis von anderen Autoimmunerkrankungen, die mit Gelenkanomalie verbunden sind, wobei:
wenn die Konzentration des Chemokins gleich dem ersten oder höher als der erste Schwellenwert ist und der Titer des Anti-CCP-Antikörpers gleich dem zweiten oder höher als der zweite Schwellenwert ist, oder die Konzentration des Chemokins gleich dem ersten oder höher als der erste Schwellenwert ist und der Titer des Anti-CCP-Antikörpers geringer als der zweite Schwellenwert ist, oder die Konzentration des Chemokins geringer als der erste Schwellenwert ist und der Titer des Anti-CCP-Antikörpers gleich dem zweiten oder höher als der zweite Schwellenwert ist, eine Information erhalten wird, die darauf hinweist, dass das Individuum wahrscheinlich rheumatoide Arthritis hat, und
wenn die Konzentration des Chemokins geringer als der erste Schwellenwert ist und der Titer des Anti-CCP-Antikörpers geringer als der zweite Schwellenwert ist, eine Information erhalten wird, die darauf hinweist, dass das Individuum wahrscheinlich keine rheumatoide Arthritis hat,
wobei das Chemokin aus MIP-1α und MIP-3α ausgewählt ist.

2. Verfahren nach Anspruch 1, wobei die anderen Autoimmunerkrankungen, die mit Gelenkanomalie verbunden sind, mindestens eine umfassen, die aus Morbus Behçet, Spondylitis ankylosans, Psoriasis-Arthritis, Polymyalgia rheumatica und adultem Morbus Still (AOSD) ausgewählt ist.

3. System, geeignet zum Liefern von Informationen zur Unterstützung der Diagnose von rheumatoider Arthritis, umfassend einen Computer, der einen Prozessor und einen von dem Prozessor gesteuerten Speicher enthält, wobei der Speicher ein Computerprogramm umfasst, das es dem Computer ermöglicht, die folgenden Schritte auszuführen:
Erhalten eines Messergebnisses von mindestens einem Chemokin und einem Anti-zyklisches-citrulliniertes-Peptid-Antikörper (Anti-CCP-Antikörper) in einer Blutprobe, die aus einem Individuum stammt, das eine Gelenkanomalie aufweist, bei einem Verdacht auf eine Autoimmunerkrankung;
Berechnen einer Konzentration des Chemokins und eines Titers des Anti-CCP-Antikörpers in der Blutprobe; und
Vergleichen der Konzentration des Chemokins mit einem ersten Schwellenwert und Vergleichen des Titers des Anti-CCP-Antikörpers mit einem zweiten Schwellenwert und Liefern einer Information, die darauf hinweist, dass das Individuum wahrscheinlich rheumatoide Arthritis hat, wenn die Konzentration des Chemokins gleich dem ersten oder höher als der erste Schwellenwert ist und der Titer des Anti-CCP-Antikörpers gleich dem zweiten oder höher als der zweite Schwellenwert ist, oder die Konzentration des Chemokins gleich dem ersten oder höher als der erste Schwellenwert ist und der Titer des Anti-CCP-Antikörpers geringer als der zweite Schwellenwert ist, oder die Konzentration des Chemokins geringer als der erste Schwellenwert ist und der Titer des Anti-CCP-Antikörpers gleich dem zweiten oder höher als der zweite Schwellenwert ist, eine Information, die darauf hinweist, dass das Individuum wahrscheinlich rheumatoide Arthritis hat, wird erhalten, oder Liefern einer Information, die darauf hinweist, dass das Individuum wahrscheinlich keine rheumatoide Arthritis hat, wird erhalten, wenn die Konzentration des Chemokins geringer als der erste Schwellenwert ist und der Titer des Anti-CCP-Antikörpers geringer als der zweite Schwellenwert ist,
wobei das Chemokin aus MIP-1α und MIP-3α ausgewählt ist.

4. System nach Anspruch 3, wobei die anderen Autoimmunerkrankungen, die mit Gelenkanomalie verbunden sind, mindestens eine umfassen, die aus Morbus Behçet, Spondylitis ankylosans, Psoriasis-Arthritis, Polymyalgia rheumatica und adultem Morbus Still (AOSD) ausgewählt ist.

5. Computerprogrammprodukt, das es einem Computer ermöglicht, das Liefern von Informationen zur Unterstützung der Diagnose von rheumatoider Arthritis auszuführen, umfassend ein computerlesbares Medium, wobei das Medium ein Computerprogramm umfasst, das es dem Computer ermöglicht, die folgenden Schritte auszuführen:
Erhalten eines Messergebnisses von mindestens einem Chemokin und einem Anti-zyklisches-citrulliniertes-Peptid-Antikörper (Anti-CCP-Antikörper) in einer Blutprobe, die aus einem Individuum stammt, das eine Gelenkanomalie aufweist, bei einem Verdacht auf eine Autoimmunerkrankung;
Berechnen einer Konzentration des Chemokins und eines Titers des Anti-CCP-Antikörpers in der Blutprobe; und
Vergleichen der Konzentration des Chemokins mit einem ersten Schwellenwert und Vergleichen des Titers des Anti-CCP-Antikörpers mit einem zweiten Schwellenwert und Liefern einer Information, die darauf hinweist, dass das Individuum wahrscheinlich rheumatoide Arthritis hat, wenn die Konzentration des Chemokins gleich dem ersten oder höher als der erste Schwellenwert ist und der Titer des Anti-CCP-Antikörpers gleich dem zweiten oder höher als der zweite Schwellenwert ist, oder die Konzentration des Chemokins gleich dem ersten oder höher als der erste Schwellenwert ist und der Titer des Anti-CCP-Antikörpers geringer als der zweite Schwellenwert ist, oder die Konzentration des Chemokins geringer als der erste Schwellenwert ist und der Titer des Anti-CCP-Antikörpers gleich dem zweiten oder höher als der zweite Schwellenwert ist, eine Information, die darauf hinweist, dass das Individuum wahrscheinlich rheumatoide Arthritis hat, wird erhalten, oder Liefern einer Information, die darauf hinweist, dass das Individuum wahrscheinlich keine rheumatoide Arthritis hat, wird erhalten, wenn die Konzentration des Chemokins geringer als der erste Schwellenwert ist und der Titer des Anti-CCP-Antikörpers geringer als der zweite Schwellenwert ist,
wobei das Chemokin aus MIP-1α und MIP-3α ausgewählt ist.

## Revendications

1. Méthode de diagnostic de la polyarthrite rhumatoïde, comprenant les étapes consistant à :
mesurer au moins une chimiokine et un anticorps anti-peptides cycliques citrullinés (anticorps anti-CCP) dans un échantillon sanguin dérivé d'un sujet présentant une anomalie articulaire et obtenir une concentration de la chimiokine et un titre de l'anticorps anti-CCP dans l'échantillon sanguin ;
comparer la concentration de la chimiokine à un premier seuil et comparer le titre de l'anticorps anti-CPP à un second seuil ; et
obtenir des informations pour distinguer la polyarthrite rhumatoïde des autres maladies auto-immunes associées à une anomalie articulaire, où :
si la concentration de la chimiokine est supérieure ou égale au premier seuil et que le titre de l'anticorps anti-CCP est supérieur ou égal au deuxième seuil, ou si la concentration de la chimiokine est supérieure ou égale au premier seuil et que le titre de l'anticorps anti-CCP est inférieur au deuxième seuil, ou si la concentration de la chimiokine est inférieure au premier seuil et le titre de l'anticorps anti-CCP est supérieur ou égal au deuxième seuil, des informations indiquant que le sujet souffre probablement de polyarthrite rhumatoïde sont obtenues et
si la concentration de la chimiokine est inférieure au premier seuil et que le titre de l'anticorps anti-CCP est inférieur au deuxième seuil, les informations indiquant que le sujet ne souffre probablement pas de polyarthrite rhumatoïde sont obtenues, où la chimiokine est choisie parmi MIP-1α et MIP-3α.

2. Méthode selon la revendication 1, où les autres maladies auto-immunes associées à une anomalie articulaire incluent au moins une de celles qui sont choisies dans le groupe constitué par syndrome de Behçet, spondylite ankylosante, arthrite psoriasique, polymyalgie rhumatismale et maladie de Still à déclenchement chez l'adulte.

3. Système adapté à l'obtention d'informations pour aider au diagnostic de la polyarthrite rhumatoïde, comprenant un ordinateur contenant un processeur et une mémoire contrôlée par le processeur, où la mémoire inclut un programme informatique permettant à l'ordinateur de mettre en oeuvre les étapes suivantes consistant à :
obtenir un résultat de mesure d'au moins une chimiokine et d'un anticorps anti-peptides cycliques citrullinés (anticorps anti-CCP) dans un échantillon sanguin dérivé d'un sujet présentant une anomalie articulaire avec soupçon de maladie auto-immune ;
calculer une concentration de la chimiokine et un titre de l'anticorps anti-CCP dans l'échantillon sanguin ; et
comparer la concentration de la chimiokine à un premier seuil et comparer le titre de l'anticorps anti-CCP à un deuxième seuil et obtenir des informations indiquant que le sujet souffre probablement de polyarthrite rhumatoïde, si la concentration de la chimiokine est supérieure ou égale au premier seuil et que le titre de l'anticorps anti-CCP est supérieur ou égal au deuxième seuil, ou si la concentration de la chimiokine est supérieure ou égale au premier seuil et que le titre de l'anticorps anti-CCP est inférieur au deuxième seuil, ou si la concentration de la chimiokine est inférieure au premier seuil et que le titre de l'anticorps anti-CCP est supérieur au deuxième seuil, des informations indiquant que le sujet souffre probablement de polyarthrite rhumatoïde sont obtenues, ou
obtenir des informations indiquant que le sujet ne souffre probablement pas de polyarthrite rhumatoïde sont obtenues, si la concentration de la chimiokine est inférieure au premier seuil et que le titre de l'anticorps anti-CCP est inférieur au deuxième seuil,
où la chimiokine est choisie parmi MIP-1α et MIP-3α.

4. Système selon la revendication 3, où les autres maladies auto-immunes associées à une anomalie articulaire incluent au moins l'une de celles qui sont choisies dans le groupe constitué par syndrome de Behçet, spondylite ankylosante, arthrite psoriasique, polymyalgie rhumatismale et maladie de Still à déclenchement chez l'adulte.

5. Produit de type programme informatique destiné à permettre à un ordinateur de mettre en oeuvre l'obtention d'informations pour aider au diagnostic de la polyarthrite rhumatoïde, comprenant un support de lecture informatique, où le support comprend un programme informatique permettant à un ordinateur de mettre en oeuvre les étapes suivantes consistant à :
obtenir un résultat de mesure d'au moins une chimiokine et d'un anticorps anti-peptides cycliques citrullinés (anticorps anti-CCP) dans un échantillon sanguin dérivé d'un sujet présentant une anomalie articulaire avec soupçon de maladie auto-immune ;
calculer une concentration de la chimiokine et un titre de l'anticorps anti-CCP dans l'échantillon sanguin ; et
comparer la concentration de la chimiokine à un premier seuil et comparer le titre de l'anticorps anti-CCP à un deuxième seuil et obtenir des informations indiquant que le sujet souffre probablement de polyarthrite rhumatoïde, si la concentration de la chimiokine est supérieure ou égale au premier seuil et que le titre de l'anticorps anti-CCP est supérieur ou égal au deuxième seuil, ou si la concentration de la chimiokine est supérieure ou égale au premier seuil et que le titre de l'anticorps anti-CCP est inférieur au deuxième seuil, ou si la concentration de la chimiokine est inférieure au premier seuil et que le titre de l'anticorps anti-CCP est supérieur au deuxième seuil, des informations indiquant que le sujet souffre probablement de polyarthrite rhumatoïde sont obtenues, ou
obtenir des informations indiquant que le sujet ne souffre probablement pas de polyarthrite rhumatoïde sont obtenues, si la concentration de la chimiokine est inférieure au premier seuil et que le titre de l'anticorps anti-CCP est inférieur au deuxième seuil,
où la chimiokine est choisie parmi MIP-1α et MIP-3α.
